# EUROPEAN PATENT APPLICATION

(11) **EP 4 166 288 A1**
(43) Date of publication of application: **19.04.2023**
(21) Application number: 21202343.6
(22) Date of filing: 13.10.2021
(51) Int. Cl.: B25J 21/00, B25J 5/00, B25J 9/00, B25J 13/02, E04B 2/74

(54) **A PREFABRICATED AUTOMATED UNMANNED AND GLOVELESS CLEANROOM PROCESSING ENCLOSURE**

(71) Applicant: Project Management Limited, Dublin 24 (IE); Pharma Integration SRL, 53100 Siena (IT)
(72) Inventor: TREACHER, Phil, Dublin, D24 XFW2 (IE); LOCK, Austin, Dublin, D24 XFW2 (IE); BECHINI, Claudio, 53100 Siena (IT)
(74) Representative: FRKelly

(57) **Abstract**

The present invention provides a prefabricated automated unmanned and gloveless cleanroom processing enclosure (10) for use, in particular but not exclusively, in the pharmaceutical and semiconductor manufacturing sectors, the enclosure defining an interior zone (24) housing at least one automated product processing module (26) and at least one remotely haptically controllable robot (34) operable within the interior zone (24) to effect interventional actions.

## Description

### Field of the invention

This invention relates to a prefabricated automated unmanned and gloveless cleanroom processing enclosure for use, in particular but not exclusively, in the pharmaceutical, medtech, or semiconductor manufacturing sectors, but has many other potential applications.

### Background of the invention

Various types of processing and/or manufacturing operations, for example in the pharmaceutical and semiconductor sectors, require extremely clean and controlled environments in order to prevent contamination of the products being produced. It is generally accepted that cleanroom personnel represent one of the main sources of potential product contamination, and thus reduction or separation of such personnel from the manufacturing process is highly desirable. In such cleanroom manufacturing operations, the two most common forms of containment systems which facilitate this personnel separation are known as restricted access barrier systems (RABS) and isolators.

RABS involve the use of a containment wall surrounding the processing equipment, such as a fillfinish station, but which remains open to the surrounding cleanroom in which the equipment is ultimately located. The containment wall includes gloves and/or ports to permit personnel to indirectly interact with the processing equipment in order to undertake any necessary interaction with the predominantly automated processes. RABS may utilise the existing air handling system of the surrounding cleanroom, or may be provided with a dedicated air handling system fully independent of the cleanroom system.

Isolators are fully enclosed and sealed environments, which permit complete isolation from the surroundings, and which can therefore offer improved protection against product contamination, and also potentially harmful exposure of the personnel in the case of hazardous materials, drugs, etc. The fully enclosed and sealed arrangement also permits extensive decontamination processes to be employed as required, for example using hydrogen peroxide or other aggressive decontaminants. Isolators again utilise gloves and/or ports to permit personnel interaction, which is generally required and often essential in order to address unexpected issues that may arise in the enclosed environment.

It is an object of the present invention to provide an alternative cleanroom processing enclosure to the above systems and which is fully automated and thus omits the requirement for any personnel interaction, but which is nevertheless capable of dealing with unexpected events in order to ensure continuity of the manufacturing or other processes conducted therein.

It is a further object of the present invention to provide a cleanroom processing enclosure which can be substantially prefabricated off site, is modular in nature, and is rapidly deployable, providing potential for use in national or global emergencies requiring low cost, low lead time and high volume emergency manufacturing capacity in order to produce critical products such as medications, vaccines or the like.

### Summary of the invention

According to the present invention there is provided a prefabricated automated unmanned and gloveless cleanroom processing enclosure comprising at least one interior zone; at least one automated product processing module located in the at least one interior zone; and at least one remotely haptically controllable robot operable within the at least one zone to effect interventional actions.

Preferably, the enclosure comprises a plurality of product processing modules.

Preferably, the enclosure comprises a plurality of remotely haptically controllable robots within the at least one zone.

Preferably, the at least one interior zone is arranged to permit locomotion of the or each remotely haptically controllable robot within the at least one interior zone.

A prefabricated automated unmanned and gloveless cleanroom processing enclosure according to any preceding claim in which the enclosure defines a plurality of interior zones.

Preferably, two or more of the interior zones are segregated from one another.

Preferably, the enclosure comprises one or more access ports in an envelope of the enclosure.

Preferably, one or more of the access ports comprises a rapid transfer port.

Preferably, one or more of the access ports are adapted for coupling with a supply container.

Preferably, the enclosure comprises one or more airlocks.

Preferably, the enclosure comprises a HVAC system operable to establish and/or regulate environmental conditions within the enclosure.

Preferably, the HVAC system comprises at least one upper air supply and at least one lower air return.

Preferably, the HVAC system comprises one or more HEPA and/or ULPA filters.

Preferably, the enclosure comprises a decontamination system operable to decontaminate the interior of the enclosure.

Preferably, the decontamination system comprises a source of decontaminant, means for injecting the decontaminant into the at least one interior zone and means for extracting the decontaminant.

Preferably, the enclosure is adapted to establish and maintain positive or negative pressurisation.

Preferably, the enclosure is adapted to establish, monitor and/or maintain cleanroom operating conditions within the one or more interior zones.

Preferably, the enclosure comprises one or more service and/or utility ports.

Preferably, one or more of the service and/or utility ports comprises a water port, electrical connector, data connector, or gas port.

Preferably, the enclosure is adapted for the modular connection to one or more duplicate and/or complimentary enclosures.

Preferably, the enclosure is adapted to be unitarily transportable via one or more modes of transport.

Preferably, the enclosure has maximum external dimensions equivalent to an intermodal transport container.

Preferably, the enclosure is prequalified.

As used herein, the term "unmanned" is intended to mean an absence, during normal operation, of human personnel in order to avoid the associated risk of contamination under normal operating conditions, but which does not exclude accessibility to personnel or other service equipment or the like to conduct non routine actions outside of normal operation.

As used herein, the term "interventional actions" is intended to mean actions or activities which fall outside of the normal operations or procedures being conducted as part of a manufacturing or processing activity, and which may from time to time be necessary in order to correct an issue in order to ensure continuity of the normal operations, and which interventional actions may be remotely effected by a human operator.

As used herein, the term "decontaminate" is intended to mean one or more steps or procedures designed to remove contaminants to a desired level and may involve physical cleaning, disinfection, chemical, thermal and/or radiation exposure or sterilisation via any suitable means.

### Brief description of the drawings

The present invention will now be described with reference to the accompanying drawings, in which:
Figure 1 illustrates a front perspective view of an exterior of a prefabricated automated unmanned and gloveless cleanroom processing enclosure according to a preferred embodiment of the present invention;
Figure 2 illustrates a rear perspective view of the prefabricated automated unmanned and gloveless cleanroom processing enclosure shown in Figure 1;
Figure 3 illustrates a front elevation of the prefabricated automated unmanned and gloveless cleanroom processing enclosure shown in Figure 1;
Figure 4 illustrates a rear elevation of the prefabricated automated unmanned and gloveless cleanroom processing enclosure shown in Figure 1;
Figure 5 illustrates a side elevation of the prefabricated automated unmanned and gloveless cleanroom processing enclosure shown in Figure 1;
Figure 6 illustrates a plan view of the prefabricated automated unmanned and gloveless cleanroom processing enclosure shown in Figure 1;
Figure 7 illustrates a sectioned plan view along line AA shown in Figure 5 and revealing the interior of the prefabricated automated unmanned and gloveless cleanroom processing enclosure;

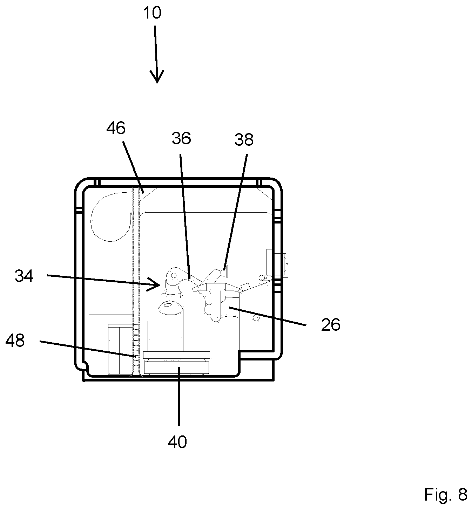

Figure 8 illustrates a sectioned side elevation along line BB shown in Figure 6 and revealing the interior of the prefabricated automated unmanned and gloveless cleanroom processing enclosure; and
Figure 9 illustrates a cut away perspective view further illustrating the interior arrangement of the of the prefabricated automated unmanned and gloveless cleanroom processing enclosure according to the invention.

### Detailed description of the drawings

Referring now to the accompanying drawings there is illustrated a prefabricated automated unmanned and gloveless cleanroom processing enclosure (hereinafter referred to as an "enclosure") according to an exemplary embodiment of the invention, and generally indicated as 10. The enclosure 10 is designed, as described in detail hereinafter, to completely eliminate the requirement for direct human interaction with the cleanroom environment established within the enclosure 10, and in a standardised form which can be substantially prefabricated off-site and easily transported as freight to enable rapid deployment. The enclosure 10 is designed to be modular in form, allowing various configurations to be achieved by connecting multiple units in a variety of configurations.

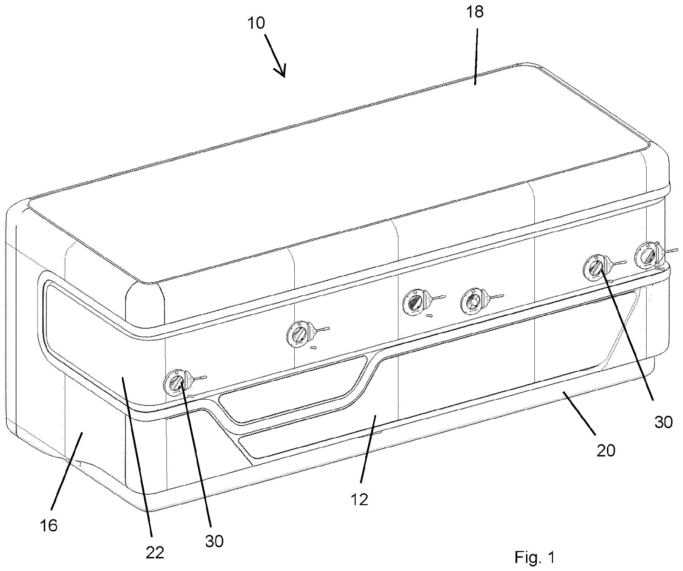

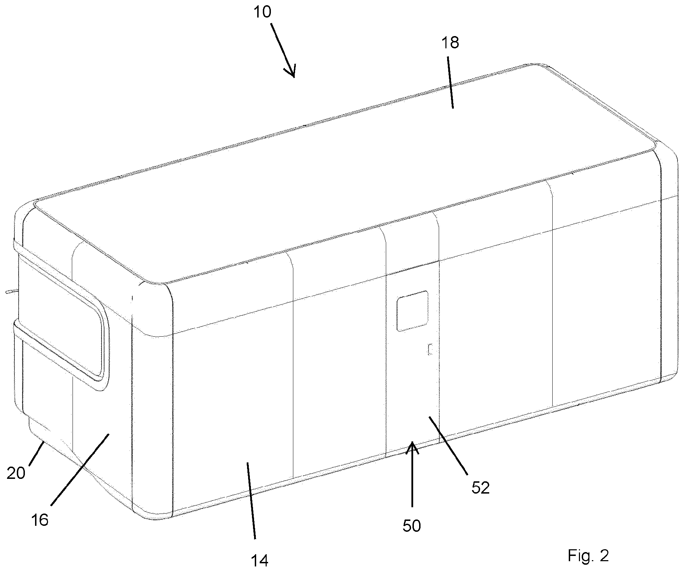

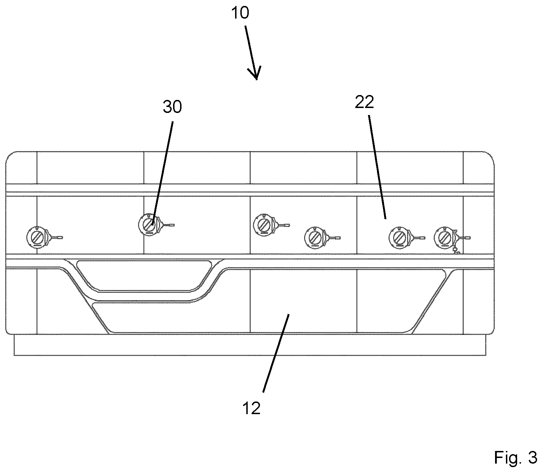

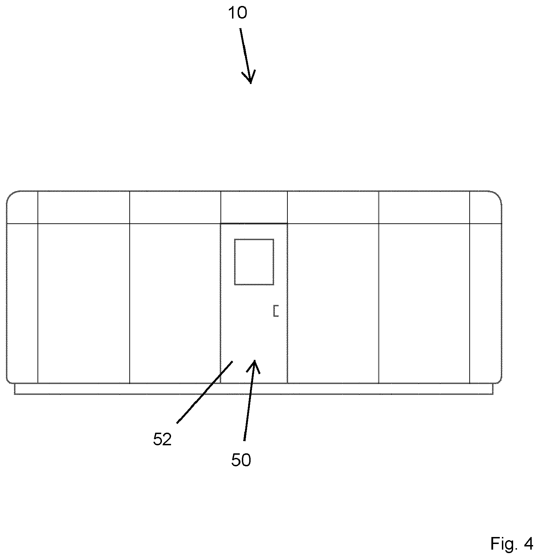

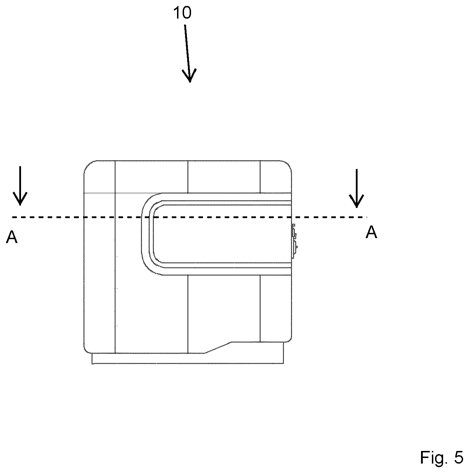

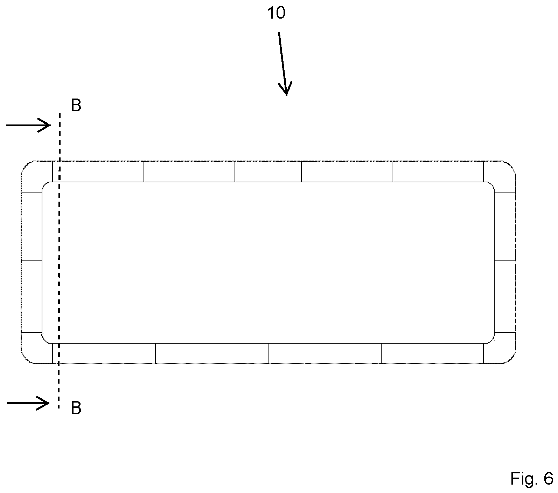

Referring in particular to Figures 1 to 6 it can be seen that the enclosure 10 is defined by a front wall 12, rear wall 14, opposed side walls 16, a roof 18 and base 20 which may act as a plinth for the enclosure 10. It will be appreciated that the construction, materials, shape and/or dimensions of the various walls of the enclosure 10 may be varied once the underlying functionality, as detailed hereinafter, is maintained. The enclosure 10 further comprises a viewing window 22 which, in the embodiment illustrated, extends laterally across the front wall 12 and wraps around onto each of the sidewalls 16. Again it will be appreciated that the shape and configuration of the window 22 may be varied, and more than one window may be provided. The window 22 may employ so called smartglass in order to allow the level of light transmissibility to be varied. This could for example allow the light transmissibility of the window 22 to be reduced to allow pulsed or UV light to be used within the enclosure 10 without affecting personnel on the exterior, and conversely to reduce the incidence of exterior light into the enclosure 10, to protect light sensitive products or the like being handled therein. It is also envisaged, in particular in view of the completely autonomous nature of the operation of the enclosure 10, that the window 22 could be omitted entirely. Although the provision of the window 22 is preferable for inspection purposes, it could be substituted or supplemented with one or more interior cameras (not shown) to allow the interior of the enclosure 10 to be monitored or viewed as required. As described in further detail hereinafter, it is preferable that the maximum external dimensions of the enclosure 10 are equivalent to an intermodal transport container (not shown) in order to allow the enclosure 10 to be transportable via one or more modes of transport, for example by rail, road, air or ocean going vessel, the enclosure 10 thus effectively functioning as a shipping container which is shaped and dimensioned to be directly handled and processed by existing freight or transport infrastructure.

The enclosure 10 is designed to house equipment for implementing one or more manufacturing or processing procedures, and preferably a sequence of manufacturing or processing modules arranged to carry out a related set of procedures, in a fully enclosed, environmentally controlled containment and in the complete absence of any human interaction whether directly or indirectly, for the reasons detailed above. The following is an exemplary and non exhaustive list of potential applications of the enclosure 10 according to the invention: aseptic filling and closing of parenteral products, handling/packing of radiopharmaceuticals, lyophilisation, medical device assembly, combination products manufacture or assembly, component assembly, electronics or semiconductor fabrication, research and development, clinical trials, commercial manufacturing, Active Pharmaceutical Incident (API) processing, and cell and gene therapy procedures. In all cases the enclosure 10 enables the above mentioned automated, unmanned cleanroom processing to be achieved, thereby omitting human interaction and thus omitting the requirement for gloved access as required with conventional RABS and isolators. The enclosure 10 is truly fully unmanned, allowing highly efficient and uninterrupted processing to be achieved.

For the purposes of the following description the configuration and operation of the enclosure 10 will be described in relation to a so called "fill and finish" procedure employed in the pharmaceutical industry in primary packaging a pharmaceutical or other medical product. It will of course be understood that this is merely for illustrative purposes and that the particular processes conducted within the enclosure 10 may be modified to suit the intended cleanroom operation.

The exemplary "fill and finish" sequence of steps may comprise a first process or step of de-nesting stacked product packaging in preparation for individual filling with the product. The next step may be the filling of each packet with the pharmaceutical product, followed by a step of stoppering or otherwise sealing the packaging, with a further step of capping the packaging, for example applying an overseal cap to the component or the like. It will be understood that this is merely an exemplary set of steps in an exemplary industrial application, and that any other procedures may be undertaken within the enclosure 10 of the invention. One or more intermediate and/or additional steps may of course be employed and undertaken. Each manufacturing/processing step may be performed at a dedication station within at least one interior zone 24 defined by the enclosure 10, with each station being defined by a dedicated processing module 26, for example a de-nesting module, a filling module, a stoppering module and a capping module. Each module 26 preferably comprises a fully automated robot adapted to the particular task and as are known in the art. Thus no further explanation of the configuration and operation of the particular modules 26 is required. The array of modules 26 are preferably arranged side by side to facilitate process flow from one station or processing module 26 to the next. The modules 26 may be pre-programmed with multiple operating protocols in order to allow quick, efficient repurposing of the enclosure 10 to handle alternative manufacturing or processing operations.

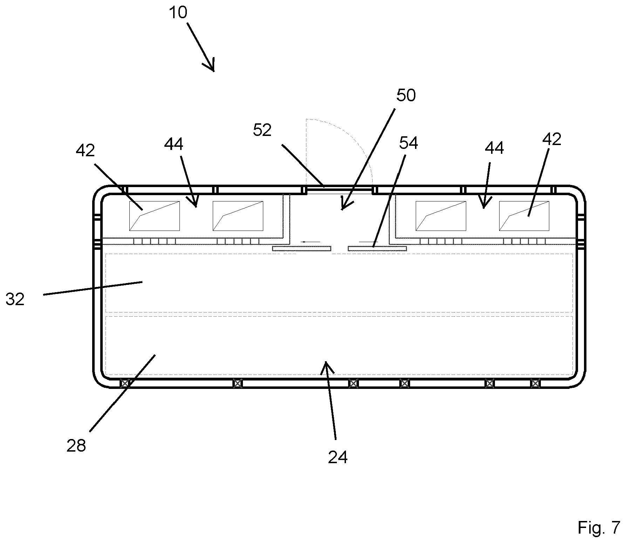

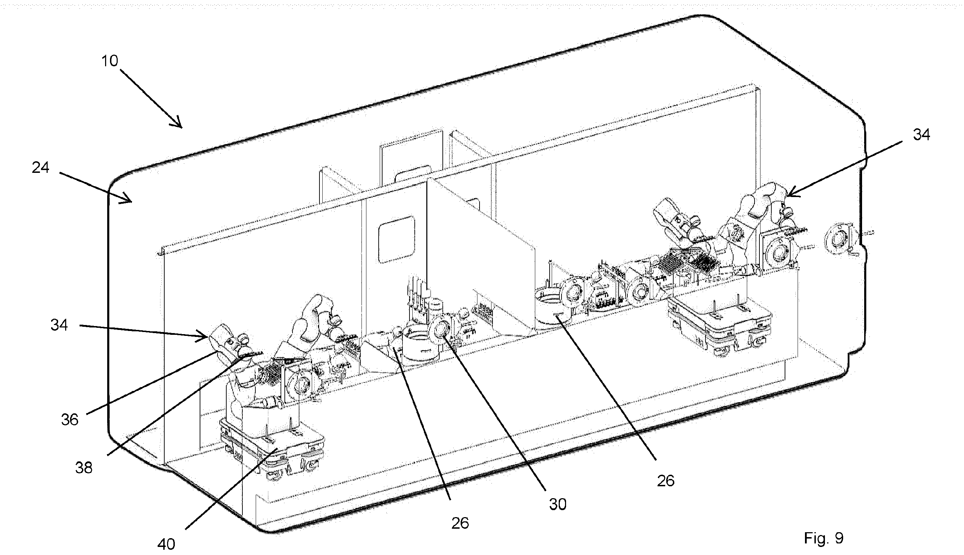

In a preferred configuration the plurality of modules 26 are arranged in a linear array adjacent the front wall 12 of the enclosure in a module zone 28 as shown in Figure 7, which forms part of the interior zone 24 and which in the embodiment illustrated extends rearwardly from the front wall 12 a distance sufficient to house the array of module 26. The module zone 28 may be a continuous uninterrupted space, or may be divided or sectioned (as shown in Figure 9), with one or more modules being located in a particular section. In order to facilitate the supply and/or extraction of the required materials or component parts to and/or from the requisite modules 26, the enclosure 10 is preferably provided with one or more ports 30, preferably conventional "rapid transfer" ports 30, which in the embodiment illustrated are located in the window 22 in front of the respective module 26, but may of course be located in any other suitable position. The ports 30 are adapted to facilitate the automated hand off of component parts to the respective module 26 while avoiding contamination, for example to facilitate the introduction of pre-sterilized product path, tools or environmental monitoring plates for use on the interior of the enclosure 10. In a particularly preferred arrangement the delivery of components to the transfer ports 30 is undertaken by suitable robots or cobots (not shown), further automating the process and reducing the risk of contamination. The ports 30 may be actuatable automatically or manually.

The enclosure 10 may also comprise a depyrogenation tunnel interface (not shown), for example in one sidewall 14, in order to facilitate the delivery into the enclosure 10 of glassware containers or the like (not shown) into which product is to be filled. Such a depyrogenation tunnel interface (not shown) is known in the art and no further explanation of the configuration and/or operation of same is therefore required.

Immediately behind the module zone 28, relative to the front wall 12, is an access zone 32 which preferably extends across the full width of the enclosure 12 from one sidewall 14 to the other, and provides an area immediately adjacent the rear of each of the modules 26, via which access to the modules 26 can be achieved. The enclosure 10 comprise at least one haptic robot 34 located in the access zone 32 and which is adapted, as described hereinafter in detail, to perform a variety of task, most notably interventional actions that may be periodically required in order to address unforeseen issues at one or more of the modules 26, thereby allowing such issues to be addressed and operations within the enclosure 10 to be continued without the local presence of human personnel.

In the embodiment illustrated the haptic robot 34 is of partial humanoid form and comprises a robotic arm 36 with an appendage 38 which may for example comprise one of a number of interchangeable alternatives, the preferred being a haptically controllable manipulator or hand (not shown). In this way a human operator may control the haptic robot 34 remotely, permitting accurate control mimicking that of a human hand/arm, and with haptic feedback/control of the gripping action, allowing a level of engagement with the modules 26 equivalent to the operator being physically present at the respective module 26.

The haptic robot 34 may be controlled by any suitable interface, for example a haptic glove (not shown) or the like, virtual reality interface or by direct vision via one or more cameras (not shown) on the haptic robot 34 and/or about the modules 26, or by means of more convention control systems. The haptic robot 34 is capable of locomotion along the access zone 32 in order to be capable of accessing each of the modules 26. In the embodiment illustrated the haptic robot 34 is mounted on a platform 40 which may be driven or otherwise displaced along the access zone 32, for example on one or more rails (not shown) or the like. Thus the operator can move the haptic robot 34 into position behind any of the modules 26, and can then use the robot arm 36 to accurately and haptically interact with the module 26, for example to reposition a wayward component or remove a broken or otherwise spoiled component, or to repair or reset the module 26 itself. It should be understood that the haptic robot 34 may be of any other suitable physical form, and for example may be effectively fully humanoid to provide increased mobility/dexterity and thus greater capability in conducting interventional actions within the enclosure 10. The haptic robot 34 may be used to deploy and otherwise interact with viable environmental monitoring, etc. as an interventional action. Such monitoring may involve active air monitoring, passive air monitoring and contact/swab monitoring. Additionally or alternatively the haptic robot 34 may support cleaning validation studies.

The haptic robot 34 is thus capable of component manipulations, solution pathway pipework manipulations, filling needle placement, and interventions for blocked component pathways. The haptic robot 34 also has the capability to transfer materials within the interior zone 24 of the enclosure 10. Additionally the haptic robot 34 may for example be utilised to affect the transfer of product or parts through the ports 30, and may be used to manually actuate the ports 30. This functionality completely removes the need for any direct human interaction with the manufacturing or processing operation, thereby eliminating the need for gloves or any other direct or physical human interface with the interior of the enclosure 10.

Furthermore, the haptic robot 34 can be utilised to implement or support in localised decontamination and/or disinfection processes to be performed within the enclosure 10, as described further hereinafter. For example the haptic robot 34 can be used to manipulate a jet wash lance, vacuum cleaning equipment or other hardware such as pulsed light sources for localised decontamination. The haptic robot 34 is capable of taking targeted samples of product for testing, arranging for the sample to be extracted from the enclosure 10 via one of the ports 30 or otherwise. The haptic robot 34 is also preferably adapted to withstand various decontamination procedures within the enclosure, and is therefore preferably resistant to routine disinfectant agents and any other decontaminants or environmental conditions that may be encountered within the enclosure 10, examples of which are detailed hereinafter. The haptic robot 34 preferably has the capability to rotate and/or otherwise be displaced during such decontamination or sterilisation processes, in order to ensure all occluded surfaces of the haptic robot 34 are exposed to the decontaminant or sterilisation agent.

In order to establish and maintain the requisite cleanroom environment, the enclosure 10 is pressurisable and in normal aseptic operations is preferably rated up to a grade A cleanroom classification, although for low bioburden operations lower grades may be sufficient depending on the operations to be performed within the enclosure 10. The enclosure 10 thus comprises a HVAC system 42 operable to establish and/or regulate environmental conditions within the enclosure 10. The HVAC system 42 is preferably located in a utility zone 44 separated and sealed from the module zone 26 and access zone 32, and in the embodiment illustrated is provided as two spaced apart utility zones 44 located adjacent the rear wall 14. The HVAC system 42 is conventional in form and operation and thus no detailed technical explanation of the configuration and operation thereof is required. In the preferred embodiment illustrated the HVAC system 42 comprises one or more air supplies or outlets 46 arranged to feed air into an upper region of the module zone 26 and/or access zone 32, and one or more air returns 48 arranged to extract air from a lower region of the module zone 26 and/or access zone 32. In this way the HVAC system 42 is operable to establish a downwardly flowing curtain or stream of air which will entrain and remove any particles or other contaminants, as is the established practice in cleanroom environments. The HVAC system 42 preferably comprises one or more HEPA and/or ULPA filters (not shown) depending on the operational requirements, in order to provide the requisite degree of filtration. In a preferred arrangement the HVAC system 42 comprises filters (not shown) at or upstream of the outlets 46 in order to filter the air entering the interior zone 24 and/or filters at or downstream of the air returns to filter the air being extracted from the interior zone 24. The enclosure 10 maybe provided with one or more sensors (not shown) in order to facilitate the desired operation of the HVAC system 42 by providing data regarding the interior and/or exterior environment for use in controlling the HVAC system 42. For example the enclosure 10 may comprise temperature sensors, humidity sensors, pressure sensors, etc. Although for the majority of processing and/or manufacturing operations the enclosure 10 will be positively pressurised, negative pressurisation may also be employed, for example for containment applications.

The enclosure 10 is further arranged to implement the autonomous decontamination of the interior of the enclosure 10, again without the presence or interaction of human personnel. The enclosure 10 therefore comprises a decontamination system (not shown) which may utilise one or more of a number of processes to achieve a desired level of decontamination. The decontamination system preferably comprises a source of decontaminant, for example nebulised or vaporised hydrogen peroxide, NOx, chlorine dioxide or other suitable decontaminating agents, which source may be contained within the enclosure 10 or may be independent of the enclosure 10 but connectable thereto by suitable means. The decontamination system preferably further comprises means for injecting the decontaminant into the at least one interior zone 24, for example an array of nozzles (not shown) distributed about the interior zone 24, and means for extracting the decontaminant after an appropriate time interval. The extraction means may comprise the HVAC system 42 or an independent gas extraction system (not shown).

The decontamination system (not shown) may comprise one or more additional elements, for example a source of radiation such as pulsed light, UV or other radiation, thermal energy, or other suitable means of decontaminating, up to and including complete sterilisation. The absence of human personnel within the enclosure 10 during normal operation greatly simplifies the procedures required for performing decontamination and if required allows decontamination to be implemented at short notice.

As detailed above, the enclosure 10 is substantially fully prefabricated off site and is both modular in form and arranged for so called "plug and play" connection to various services and/or utilities, for example water, power, data, process air, nitrogen or other gases, and decontaminants such as gaseous hydrogen peroxide or the like. The enclosure 10 is therefore preferably provided with one or more service and/or utility ports (not shown) of conventional form, in order to allow connection to the above mentioned services and/or utilities. In this way one or more of the enclosures 10 can be transported to a deployment site, for example within an exiting cleanroom facility, and the various services and utilities connected thereto with ease, allowing the enclosure to be operation within a very short lead time. One or more of the enclosures 10 may be connected together in any required configuration in order to provide the necessary work flow to achieve the required processing and/or manufacturing operation. This modular design also enables additional enclosures 10 to be added over time, providing scalability. Where multiple modules 10 are interconnected, one or more of the haptic robots 34 or other robot or vehicle (not shown) may be capable of moving from one enclosure 10 to another. In such a situation is preferably that the haptic robot 34 is subject to decontamination when transferring from one enclosure 10 to another, which could be undertaken in the airlock 50 or another dedicated transitional zone (not shown) forming a bridge between the connected enclosures 10.

As noted above, the enclosure 10 may comprise a depyrogenation tunnel interface (not shown), for example in one sidewall 14. Similarly the enclosure 10 may be provided with additional interfaces (not shown) to permit connection to additional applications such as a washer tunnel, E-beam, a debagger, a packaging line, an external surface component washer, labelling equipment, etc.

Although the enclosure 10 is designed to operate in the complete absence of human personnel within the enclosure 10, an airlock such as a personnel airlock 50 to enable human or robotic access to the interior of the enclosure 10 outside of normal operation is preferably provided. In the embodiment illustrated the airlock 50 is located in the rear wall 14 between the pair of utility zones 44, but it will be understood that any other suitable location may be selected. The airlock 50 comprises an outer door 52 and an inner door 54 defining an airlock therebetween. The inner door 54 may comprises a set of double doors, for example where the access zone 32 is separated into two sections, with one of each of the double doors providing access to either section of the access zone 32, as illustrated for example in Figure 9. Where such a floor plan is adopted, the enclosure 10 is preferably provided with a pair of the haptic robots 34, one located in each section of the access zone 32 with access to each module 26 within that section as hereinbefore described. The airlock 50 allows a human or robot to access the interior of the enclosure 10 as may be required, for example to implement a reconfiguration of the interior layout, replacing or updating modules 26 and/or other equipment, or for any other purposes.

The enclosure 10 of the present invention, by including the haptic robot 34, completely eliminates the need for direct human interaction with the conditioned environment in which the uninterrupted contaminant free processing and/or manufacturing can therefore take place. The prefabricated form significantly reduces cost and enables very short lead times from order to installation of the enclosure 10, allow the rapid and large scale production of product, for example urgently required medical products such as vaccines or the like.

The self contained form of the enclosure 10 enables full testing and qualification prior to delivery/installation, further reducing the lead time to operational status, with all automation of modules 26 and/or other on-board systems pre-tested. To further reduce lead time, in a particularly preferred embodiment the enclosure 10 is adapted to be directly transportable via one or more modes of transport without requiring any additional significant packaging or containment. For example the enclosure 10 may be dimensioned to approximate a conventional intermodal transport container, the walls 12, 14 16, 18, 20 having the necessary structural integrity to allow the enclosure 10 to be directly handled and processed by existing freight or transport infrastructure, further reducing the lead times from order placement to delivery and ultimately installation on site.

The design and operation of the enclosure 10 greatly expands the locations/sites at which such processing and/or manufacturing operations can be undertaking, allowing for localised production at the so called "point of care". This avoids the requirement for shipping large volumes of high value components or constituents of the product to be produced, and allows products to be tailored to the needs of the individual locality, region or country. Local and/or national government can then be in direct control of the local supply of the products produced, avoiding reliance on outside agencies.

The enclosure 10 thus provides a significant number of benefits over conventional cleanroom RABS and isolators. The enclosure 10 enables a modular cleanroom facility design of a standardised form and which has plug and play capabilities for rapid connection to the necessary services and utilities required for operation. The enclosure 10 may be delivered fully fitted with the necessary processing and/or manufacturing modules 26 installed, is scalable and effectively infinitely configurable, while being robust, rapidly deployable and providing a human free environment to dramatically reduce the potential for contamination. The humanless environment established by the enclosure 10 also provides an increased level of assurance for extended production runs, batches, or number of batches without the need for decontamination.

## Claims

1. A prefabricated automated unmanned and gloveless cleanroom processing enclosure comprising at least one interior zone; at least one automated product processing module located in the at least one interior zone; and at least one remotely haptically controllable robot operable within the at least one zone to effect interventional actions.

2. A prefabricated automated unmanned and gloveless cleanroom processing enclosure according to claim 1 comprising a plurality of product processing modules.

3. A prefabricated automated unmanned and gloveless cleanroom processing enclosure according to claim 1 or 2 comprising a plurality of remotely haptically controllable robots within the at least one zone.

4. A prefabricated automated unmanned and gloveless cleanroom processing enclosure according to any preceding claim in which the at least one interior zone is arranged to permit locomotion of the or each remotely haptically controllable robot within the at least one interior zone.

5. A prefabricated automated unmanned and gloveless cleanroom processing enclosure according to any preceding claim in which the enclosure defines a plurality of interior zones.

6. A prefabricated automated unmanned and gloveless cleanroom processing enclosure according to claim 5 in which two or more of the interior zones are segregated from one another.

7. A prefabricated automated unmanned and gloveless cleanroom processing enclosure according to any preceding claim comprising one or more access ports in an envelope of the enclosure.

8. A prefabricated automated unmanned and gloveless cleanroom processing enclosure according to claim 7 in which one or more of the access ports comprises a rapid transfer port.

9. A prefabricated automated unmanned and gloveless cleanroom processing enclosure according to claim 7 or 8 in which one or more of the access ports are adapted for coupling with a supply container.

10. A prefabricated automated unmanned and gloveless cleanroom processing enclosure according to any preceding claim comprising one or more airlocks.

11. A prefabricated automated unmanned and gloveless cleanroom processing enclosure according to any preceding claim comprising a HVAC system operable to establish and/or regulate environmental conditions within the enclosure.

12. A prefabricated automated unmanned and gloveless cleanroom processing enclosure according to claim 11 in which the HVAC system comprises at least one upper air supply and at least one lower air return.

13. A prefabricated automated unmanned and gloveless cleanroom processing enclosure according to claim 11 or 12 in which the HVAC system comprises one or more HEPA and/or ULPA filters.

14. A prefabricated automated unmanned and gloveless cleanroom processing enclosure according to any preceding claim comprising a decontamination system operable to decontaminate the interior of the enclosure.

15. A prefabricated automated unmanned and gloveless cleanroom processing enclosure according to claim 13 in which the decontamination system comprises a source of decontaminant, means for injecting the decontaminant into the at least one interior zone and means for extracting the decontaminant.

16. A prefabricated automated unmanned and gloveless cleanroom processing enclosure according to any preceding claim in which the enclosure is adapted to establish and maintain positive or negative pressurisation.

17. A prefabricated automated unmanned and gloveless cleanroom processing enclosure according to any preceding claim in which the enclosure is adapted to establish, monitor and/or maintain cleanroom operating conditions within the one or more interior zones.

18. A prefabricated automated unmanned and gloveless cleanroom processing enclosure according to any preceding claim comprising one or more service and/or utility ports.

19. A prefabricated automated unmanned and gloveless cleanroom processing enclosure according to claim 17 in which one or more of the service and/or utility ports comprises a water port, electrical connector, data connector, or gas port.

20. A prefabricated automated unmanned and gloveless cleanroom processing enclosure according to any preceding claim in which the enclosure is adapted for the modular connection to one or more duplicate and/or complimentary enclosures.

21. A prefabricated automated unmanned and gloveless cleanroom processing enclosure according to any preceding claim in which the enclosure is adapted to be unitarily transportable via one or more modes of transport.

22. A prefabricated automated unmanned and gloveless cleanroom processing enclosure according to any preceding claim in which the enclosure has maximum external dimensions equivalent to an intermodal transport container.

23. A prefabricated automated unmanned and gloveless cleanroom processing enclosure according to any preceding claim in which the enclosure is prequalified.
